# EUROPEAN PATENT SPECIFICATION

(11) **EP 1 257 202 B1**
(45) Date of publication and mention of the grant of the patent: **30.03.2005**
(21) Application number: 01908526.5
(22) Date of filing: 15.02.2001
(51) Int. Cl.: A61B 5/18, B60K 28/06

(54) **METHOD AND MEANS FOR MONITORING DRIVER ALERTNESS**
VERFAHREN UND GERÄT ZUR ÜBERWACHUNG DER AUFMERKSAMKEIT DES FAHRERS
PROCEDE ET ORGANE PERMETTANT DE SURVEILLER LA VIVACITE D'UN CONDUCTEUR

(30) Priority: 15.02.2000 SE 0000489; 20.06.2000 SE 0002308
(43) Date of publication of application: 20.11.2002
(73) Proprietor: CESIUM AB, 641 22 Katrineholm (SE)
(72) Inventor: BJÖRKMAN, Mats, S-442 97 Kode (SE)
(74) Representative: Eriksson, Hans Gustaf
(86) International application number: PCT/SE2001/000334
(87) International publication number: WO 2001/060254

(56) References cited:
- WO-A1-00/31712
- WO-A1-96/16830
- WO-A1-97/46158
- US-A- 3 611 344

## Description

The present invention relates to a method and an arrangement for imparting an impulse to a vehicle whilst underway, the impulse demanding a spontaneous reaction from the driver.

The ultimate object of the invention is to prevent accidents and near-accidents in traffic by detecting and drawing attention to hazardous driver behaviour caused, for example, by alcohol, drugs or fatigue before it results in accidents.

Substances that have an influence onto the central nervous system, such as alcohol and drugs affect the human autonomic nervous system in such a way that skills associated with trained reflexes and/or conditioned response behaviour become slower and more unsure. In order for a vehicle to be driven safely, the driver must react quickly to impressions and the performance of the vehicle. In conditioned response behaviour the reaction time is as little as 50 ms for a trained and alert driver.

By applying a stimulus, such as a very slight steering deflection, to the vehicle while it is underway and then measuring, by way of the steering wheel, the time that elapses before the initiation of a reflex-action compensatory movement on the part of the driver, and possibly also the force that is exerted on the steering wheel, conclusions may be drawn regarding the driver's alertness and capacity to handle the vehicle.

A delayed reaction may be caused by the effect of drugs. The stimulus or impulse may be imparted to the steering and the corrective reaction by the driver measured by way of time and possibly g-force sensors. Since the time at which the stimulus was imparted is known, it is easy to measure the reaction time delay. In the event of uncertain results a series of well-timed stimuli can be imparted for more precise monitoring of the driver's frequently subliminal reaction.

A driver recording can also be made in connection with driving instruction at driving schools for better adaptation of driver training to the individual's requirements and level of competence attained at any given time.

The invention will be described below in relation to an exemplary embodiment shown, in which:
Fig. 1 shows a diagram illustrating vehicle movement (steering manipulation) and the driver's steering wheel deflection, in which deflection (g-forces) is recorded on the ordinate and time on the abscissa,
Fig. 2 shows a schematic diagram of a torque registering arrangement in a steering wheel shaft, for example, and
Fig. 3 shows a schematic view from above of a vehicle and the location of the equipment that is required for implementing the invention.

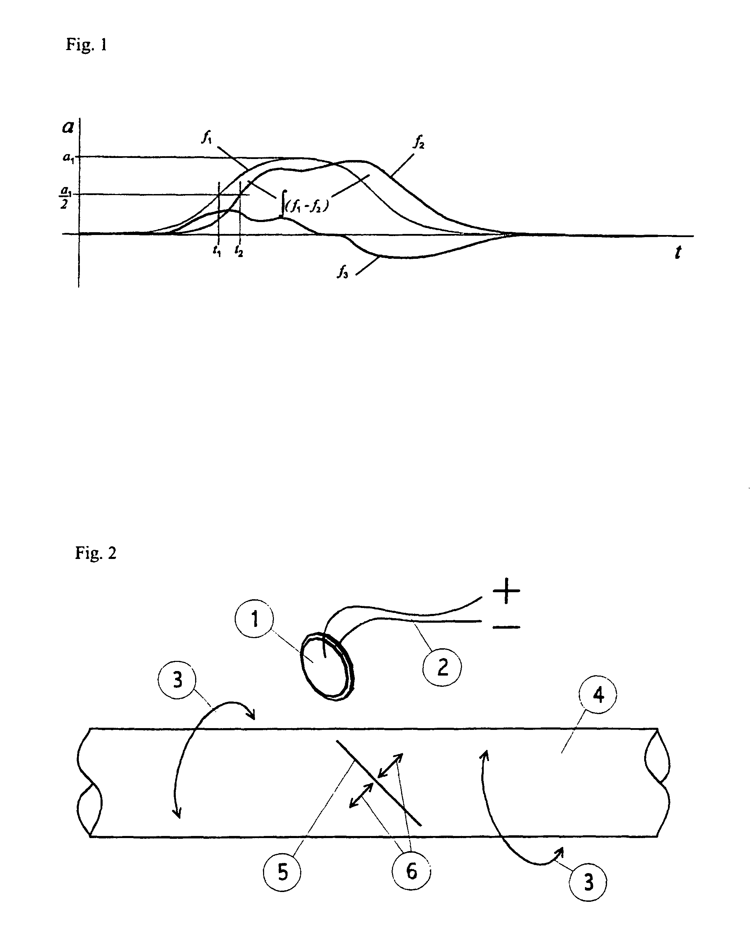

The graphs in Figure 1 describe a situation in which the vehicle has been acted upon either by an irregularity in the road surface or by a manipulator device, which has acted upon the vehicle's steering. It can be seen that the driver's graph *f*2 "lags behind" the vehicle's (the steering's) graph *f*1 and this is due to the fact that it takes a certain time for the driver to react. The ability of the driver to compensate for an influence from *f*1 can be detected in at least two ways:

By integrating the difference between the graphs *f*1 and *f*2 we arrive at the shaded area in the figure. The faster and better the driver compensates the smaller the surface will be. The area of the surface is varied over time and the shape of the variation shows the driver's flexibility in compensating.

The amplitude of the g-force in a lateral direction *f*3(t) provides a measure of the driver's reaction time and the shape of the curve shows the movement pattern. Jerky movements show up directly.

In addition any overreaction in compensatory steering deflection can also be registered. There is a risk that an overreaction in compensatory steering deflection will give rise to vehicle behaviour that is difficult to control.

The reaction time can be calculated as *t*2 - *t*1 at a predetermined level, for example half the maximum value of *f*1, or be assessing the amplitude of *f*3 in relation to *f*1.
*f*1 represents the torque acting on the steering column from the vehicle side (the influence of the manipulator device)
*f*2 is the torque which the driver exerts on the steering column in order to compensate for *f*1 (signal from the torque sensor)
*f*3 shows the resultant laterally acting g-forces on the vehicle (signal from g-force sensor)

Axis *a* is amplitude and axis *t* is the time.
*a*1 is the maximum of *f*1 during a cycle.

The driver's reaction time can be calculated as *t*2 - *t*1.

The shaded area can be seen as a measure of the driver's ability to compensate for the influence of the vehicle or the manipulator device on the steering column, smaller area = good compensation, larger area = poorer compensation. Any tendency to overreaction can also be reliably revealed.

Fig. 2 shows the torque registering arrangement comprising a piezo element 1 of standard type, connecting wires 2, a shaft 4, a slot 5 for fitting a sensor. A torsional force 3 is applied to the shaft 4, and 6 indicates converted forces. There is provided a steering column with diagonal slot, which converts the torsional force into a linear, perpendicular force. Depending on the type of force measurement desired, that is to say whether the force is a static or dynamic force, various sensors can be located in the slot. If the force is a dynamic or pseudo-dynamic force a standard piezo element is eminently suitable. The arrangement converts the torsional force or torque into an electrical voltage. Owing to the self-discharge of the piezo element the lower frequency limit is in the order of magnitude of 0.1 Hz. The piezo element cannot be used for measuring static forces. In order that the shaft may not loose its stability on the arrangement, the slot is suitably covered by an outer protective tube, which functions both as mechanical stabilisation and as protection against dirt and moisture. Outer protective tubes are not shown in the drawing.

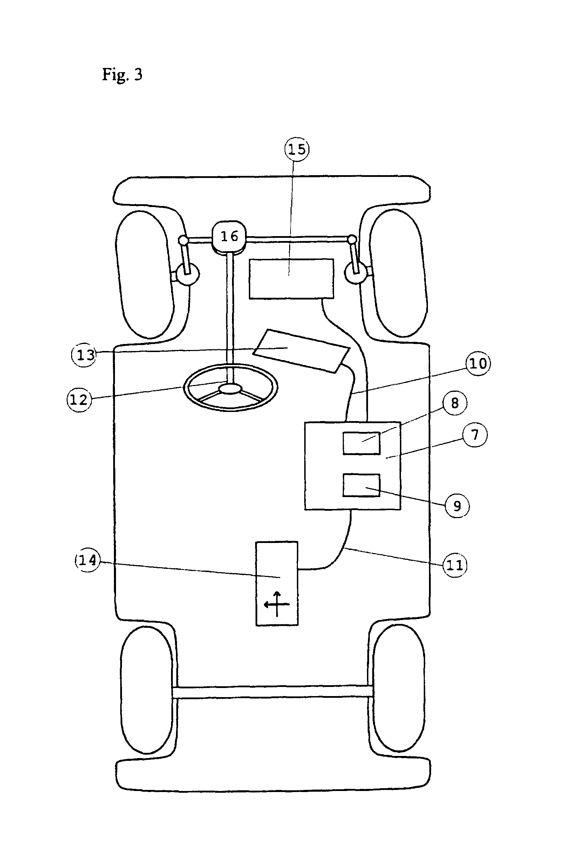

Fig. 3 shows a schematic view from above of a vehicle equipped for performing the method according to the invention, in which 7 denotes a computer module with central processing unit and memory, 8 a processing unit or data processing equipment, for example a microprocessor, 9 a data memory, 10 a communications interface to a display unit, 11 an interface from the g-force sensor, 12 interface for the manipulation device to the power-assisted steering on a car, for example, 13 a display unit with audible, visual and/or other means of communication suited to the application, 14 a g-force sensor, 15 a manipulation device for the vehicle steering, for example and 16 a torque and/or angular velocity-registering device for the steering column of a vehicle.

The object of the invention is to induce a measuring impulse that runs through the driver-vehicle network with the aim of mapping the degree of feedback in the "driver/vehicle" system. This system includes the driver's brain with motor and sensory functions, the dynamic characteristics of the vehicle and the characteristics of the road, together with the interfaces between them.

In order to get a vehicle driver to adapt to his or her own instantaneous capacity as driver and to the vehicle and to the road conditions in which they are travelling, it is extremely important that the technology that will form the basis for decisions on a warning of unsuitable behaviour be based on sound principles. This adaptation will most preferably be made voluntarily. If this is not the case it may be necessary to automatically limit the vehicle speed, for example, it then being even more important that a correct basis be identified for deciding this.

The present invention relates to technology which further increases the scope for precisely determining the instantaneous presence of mind of the driver even where a reduced margin of safety is indicated.

The result of mapping is used to determine:
a) the driver's attentiveness/presence of mind. Requires comparison with standard.
b) the level of learning attained by the driver. Requires analysis of change in the measured values over time.
c) dynamic characteristics of the vehicle.

The invention is not confined to the exemplary embodiments specified above, but may lend itself to modifications without departing from the scope of the claims specified below.

## Claims

1. Method for imparting an impulse to a vehicle whilst underway by way of one or more actuators, the driver normally reacting spontaneously and subliminally to the impulse, **characterised in that** the impulse is imparted to the steering and that the driver's response is detected by one or more sensors, for example steering angle sensor, torque sensor, g-force sensor, eye movement sensor, etc.

2. Method according to Claim 1, **characterised in that** impulse and response are compared in order to draw conclusions regarding the driver's presence of mind, which stands in proportion to the difference between impulse and response.

3. Method according to Claim 1 or 2, **characterised in that** the impulses are varied in time, amplitude and form in order to numerically map the personal profile of a particular driver.

4. Method according to any of the preceding Claims, **characterised in that** the personal profile for a specific driver in respect of steering wheel deflection and g-forces occurring is fixed beforehand and is stored on an electronic storage medium and compared with earlier profiles in order thereby to monitor deviations, or for use as reference in driving practice and the development of driving proficiency.

5. Method according to any of the preceding Claims, **characterised in that** the accuracy of the comparisons between impulse and response is improved by means of repeated measurements and mathematical operations, such as averaging.

6. Method according to any of the preceding Claims, **characterised in that** the impulses are varied in time, amplitude and form in order to disguise them from the driver, so as to be able to carry out continuous measurements.

7. Method according to any of the preceding Claims, **characterised in that** the impulse or impulses are disguised by using the driver's own steering wheel movements as trigger signal in order to thereby utilise the driver's activated feedback system.

8. Method according to any of the preceding Claims, **characterised in that** the impulse is imparted to the vehicle by way of an actuator acting on the steering system, preferably the vehicle's existing power-assisted steering.

9. Arrangement for generating an impulse for a vehicle whilst underway by way of one or more actuators, the driver normally reacting spontaneously and subliminally to the impulse, and for performing the method according to Claim 1, **characterised in that** a microprocessor unit with software that generates impulses for the vehicle, registers sensor signals and processes, compares and stores impulses and responses.

10. Arrangement according to Claim 9, **characterised in that** a sensor for registering pressure stresses e.g. torque, is arranged in connection with the vehicle steering, preferably in the steering column in a slot reserved for this purpose, which is of dimensions such that the slot opening is increased or reduced as a function of the instantaneous change and magnitude of the torque applied to the steering wheel.

## Patentansprüche

1. Verfahren zur Übermittlung eines Impulses an ein Fahrzeug während der Fahrt mittels eines oder mehrerer Auslöser, wobei der Fahrer auf den Impuls üblicherweise spontan und unterbewusst reagiert, **dadurch gekennzeichnet, dass** der Impuls auf die Steuerung (oder auf ein anderes Teil mit welchem der Fahrer üblicherweise in Kontakt ist) übermittelt wird, und dass Fahrers Antwort mittels eines oder mehrerer Sensoren, beispielsweise Lenkwinkel-, Drehmoment-, Beschleunigungskraft-, Augenbewegungssensor usw. detektiert wird.

2. Verfahren nach Anspruch 1, **dadurch gekennzeichnet, dass** der Impuls und die Antwort verglichen werden, um Schlüsse bezüglich Fahrers Geistesgegenwart zu ziehen, welche Geistesgegenwart dem Unterschied zwischen dem Impuls und der Antwort proportional ist.

3. Verfahren nach einem der Ansprüche 1 oder 2, **dadurch gekennzeichnet, dass** die Impulse zur numerischen Ausarbeitung des persönlichen Profils eines spezifischen Fahrers in Zeit, Amplitude und Form geändert werden.

4. Verfahren nach einem der vorangehenden Ansprüche, **dadurch gekennzeichnet, dass** das persönliche Profil des spezifischen Fahrers bezüglich Ausschlag des Lenkrads und auftretende Beschleunigungskräfte zuvor bestimmt, auf einem elektronischen Speichermedium gespeichert und mit früheren Profilen verglichen wird zum Überprüfen von Abweichungen oder Gebrauch als Referenz für die Fahrpraxis und Entwicklung von Fahrfähigkeiten.

5. Verfahren nach einem der vorangehenden Ansprüche, **dadurch gekennzeichnet, dass** die Genauigkeit der Vergleiche zwischen dem Impuls und der Antwort durch wiederholte Messungen und mathematische Operationen wie Durchschnittsberechnung erhöht wird.

6. Verfahren nach einem der vorangehenden Ansprüche, **dadurch gekennzeichnet, dass** die Impulse zum Tarnen vor dem Fahrer in Zeit, Amplitude und Form geändert werden, so dass stetige Messungen ermöglicht werden.

7. Verfahren nach einem der vorangehenden Ansprüche, **dadurch gekennzeichnet, dass** der Impuls oder die Impulse getarnt werden, indem Fahrers eigene Lenkradbewegungen als Auslösesignal zur Verwendung des aktivierten Feedback-Systems des Fahrers verwendet werden.

8. Verfahren nach einem der vorangehenden Ansprüche, **dadurch gekennzeichnet, dass** der Impuls an das Fahrzeug mittels eines Auslösers übertragen wird, wobei der Auslöser auf das Lenksystem, vorzugsweise auf die bestehende kraftunterstützte Lenkung des Fahrzeugs einwirkt.

9. Einrichtung zur Erzeugung eines Impulses für ein Fahrzeug in Fahrt mittels eines oder mehrerer Auslöser, wobei der Fahrer auf den Impuls üblicherweise spontan und unterbewusst reagiert, und zur Ausführung des Verfahrens nach Anspruch 1, **dadurch gekennzeichnet, dass** eine Mikroprozessoreinheit mit Software, die Impulse für das Fahrzeug erzeugt, Sensorsignale registriert, sowie Impulse und Antworten verarbeitet, vergleicht und abspeichert.

10. Einrichtung nach Anspruch 9, **dadurch gekennzeichnet, dass** ein Sensor zur Registrierung von Druckbelastung, beispielsweise Drehmoment in Verbindung mit der Lenkung eingerichtet ist, vorzugsweise in der Lenksäule in einem für diesen Zweck vorgesehenen Schlitz, dessen Masse so gewählt sind, dass die Schlitzöffnung in Funktion von der momentanen Änderung und Grösse des Drehmoments einwirkend auf das Lenkrad vergrössert oder verkleinert wird.

## Revendications

1. Procédé pour donner une impulsion à un véhicule pendant qu'il roule au moyen d'un ou plusieurs organes d'actionnement, le conducteur réagissant normalement spontanément et de façon subliminale à l'impulsion, **caractérisé en ce que** l'impulsion est donnée au volant et **en ce que** la réponse du conducteur est détectée par un ou plusieurs capteurs, par exemple, un capteur d'angle de conduite, un capteur de couple, un détecteur de gravité, un capteur de mouvement des yeux, etc.

2. Procédé selon la revendication 1, **caractérisé en ce que** l'impulsion et la réponse sont comparées afin de tirer des conclusions concernant la présence d'esprit du conducteur, qui est proportionnelle à la différence entre l'impulsion et la réponse.

3. Procédé selon la revendication 1 ou 2, **caractérisé en ce que** les impulsions varient dans le temps, en amplitude et en forme, afin d'établir une carte numérique du profil personnel d'un conducteur particulier.

4. Procédé selon l'une quelconque des revendications précédentes, **caractérisé en ce que** le profil personnel d'un conducteur particulier en ce qui concerne la déviation du volant et les forces d'accélération est fixé à l'avance et est stocké sur un support de stockage électronique et comparé à des profils antérieurs afin de surveiller ainsi les déviations, ou pour être utilisé comme référence dans la pratique de la conduite et le développement d'une aptitude à la de conduite.

5. Procédé selon l'une quelconque des revendications précédentes, **caractérisé en ce que** la précision des comparaisons entre l'impulsion et la réponse est améliorée au moyen de mesures répétées et d'opérations mathématiques, telles que le calcul d'une moyenne.

6. Procédé selon l'une quelconque des revendications précédentes, **caractérisé en ce que** les impulsions varient dans le temps, en amplitude et en forme afin de les dissimuler au conducteur, de façon à permettre d'effectuer des mesures continues.

7. Procédé selon l'une quelconque des revendications précédentes, **caractérisé en ce que** la ou les impulsions sont dissimulées en utilisant les propres mouvements de volant du conducteur comme signal de déclenchement afin d'utiliser ainsi le système à circuits rétroactifs activé du conducteur.

8. Procédé selon l'une quelconque des revendications précédentes, **caractérisé en ce que** l'impulsion est donnée au véhicule au moyen d'un organe d'actionnement agissant sur le système de conduite, de préférence la conduite assistée existante du véhicule.

9. Agencement pour générer une impulsion pour un véhicule pendant qu'il roule au moyen d'un ou plusieurs organes d'actionnement, le conducteur réagissant normalement spontanément et de façon subliminale à l'impulsion, et pour mettre en oeuvre le procédé selon la revendication 1, **caractérisé en ce qu**'une unité à microprocesseur avec un logiciel qui génère des impulsions pour le véhicule, enregistre des signaux de capteur et traite, compare et stocke des impulsions et des réponses.

10. Agencement selon la revendication 9, **caractérisé en ce qu'**un capteur d'enregistrement des forces de pression (par exemple, un couple) est agencé en liaison avec la conduite du véhicule, de préférence dans la colonne de direction dans une rainure réservée à cet effet, qui a des dimensions telles que l'ouverture de la rainure est augmentée ou réduite en fonction du changement instantané et de l'amplitude du couple appliqué au volant.
